# EUROPÄISCHE PATENTANMELDUNG

(11) **EP 2 014 759 A1**
(43) Veröffentlichungstag der Anmeldung: **14.01.2009**
(21) Anmeldenummer: 08400019.9
(22) Anmeldetag: 31.03.2008
(51) Int. Cl.: C12M 1/107

(54) **Präparat zur Optimierung der Methangas-Bildung in Biogasanlagen**

(30) Priorität: 21.06.2007 DE 102007029102
(71) Anmelder: Tilco Biochemie GmbH, 23858 Reinfeld (DE)
(72) Erfinder: Scharafat , Iradj, 23843 Travenbrück (DE)
(74) Vertreter: Klickow, Hans-Henning

(57) **Zusammenfassung**

Das Präparat dient zur Optimierung der Methangasbildung in Biogasanlagen und weist mindestens eine erste organische Komponente sowie mindestens eine zweite anorganische Komponente auf. Die erste Komponente besteht aus Algenmaterial.

## Beschreibung

Die Erfindung betrifft ein Präparat zur Optimierung der Methangasbildung in Biogasanlagen, das mindestens eine erste organische Komponente sowie mindestens eine zweite anorganische Komponente aufweist.

In Biogasanlagen werden organische Stoffe durch Einwirkung von Bakterien zersetzt. Bei Verwendung geigneter Bakterien entsteht hierbei Methangas, das üblicherweise einem Generator zur Erzeugung elektrischer Energie zugeführt wird, die in ein Versorgungsnetz eingespeist wird.

Der Wirkungsgrad bei der Methangaserzeugung hängt von einer Vielzahl von Faktoren ab. Ein wesentlicher Einflußfaktor ist hierbei die Vermehrungsrate der Nutzbakterien. Ein wesentliches Problem besteht darin, daß in der Biomasse eine Vielzahl von Bakterien enthalten sind, die miteinander konkurrieren.

Es ist bereits bekannt, der zu zersetzenden Biomasse geeignete Präparate hinzuzusetzen, um die Vermehrung der Nutzbakterien zu fördern. Sowohl die Wirksamkeit der bekannten Präparate als auch die erforderliche Präparatmenge je Tonne Biomaterial wird jedoch als unbefriedigend empfunden.

Aufgabe der vorliegenden Erfindung ist es daher, ein Präparat der einleitend genannten Art derart zu verbessern, daß mit einer vergleichsweise geringen Präparatmenge die Vermehrung der Nutzbakterien unterstützt wird.

Diese Aufgabe wird erfindungsgemäß dadurch gelöst, daß die erste Komponente aus Algenmaterial besteht.

Erfindungsgemäß wurde herausgefunden, daß ein Zusatz von Algenmaterial zur Biomasse innerhalb des Biogasreaktors zu einer optimalen Umgebung für die Nutzbakterien führt. Das Algenmaterial dient hierbei sowohl als zusätzliche Nahrung für die Bakterien und führt darüber hinaus zu optimalen Umgebungsbedingungen. Darüber hinaus wird die Vermehrung der nicht als Nutzbakterien angesehenen weiteren Bakterien gehemmt.

Es zeigt sich, daß der Zusatz des Algenmaterials insbesondere zu einem Beginn der Biogasproduktion von erheblichem Vorteil ist, da für die Nutzbakterien hierdurch gegenüber den weiteren Bakterien optimierte Vermehrungsbedingungen bereitgestellt werden. Nach einer entsprechenden Initialzündung hat das Algenmaterial zwar weiterhin eine positive Wirkung auf die Vermehrung der Bakterien, diese sind jedoch bereits derart gestärkt, daß deutliche Vermehrungsvorteile gegenüber den weiteren Bakterien vorliegen.

Nach der entsprechenden Initialzündung besteht ein wesentlicher Teil der fördernden Wirkung des Algenmaterials in einer Optimierung des Stoffwechsels der Nutzbakterien und einer hierdurch gesteigerten Produktion an Biogas.

Eine erste Komponente wird auf Basis von Algenmaterial hergestellt. Insbesondere ist ein aufgeschlossenes Algenmaterial geeignet.

Der Algen-Aufschluss ist ein Feucht- und (oder) Flüssig-Aufschluss mit Algen, Wasser und Ammonium, Erdalkali- und Alkalimetallen.

Das ziel ist, das gesamte Algenmaterial (als Rohalgen oder Algenmehle) mit Alkalimetallen wie Kalium, Natrium, Ammonium, Magnesium usw. mit Wasserzusatz so aufzuschließen, dass ein granuliertes Produkt, das auch Alginate enthält, entsteht. Reine Kalium-, Natrium-, Ammonium- oder Magnesiumalginate sind auch zweckmäßig und können auch verwendet werden. Das gesamte Algenmaterial mit Alkalisalzen und Wasser muss unter sanftem Aufschluss so behandelt werden, dass die Inhaltsstoffe der Algen verfügbar sind und nicht verloren gehen dürfen. Nicht nur Alginate sind in diesen Verfahren wichtig, sondern auch Aminosäuren, Vitamine, Hormone, Laminarin, Focucin, Betain und viele andere pflanzliche Stoffe.

Insbesondere eine flüssige, dickflüssige, zähflüssige, pulverförmige oder granulierte Form der aufgeschlossenen Braunalgen ist in der Lage, den Methan- und Biogas-Bakterien als Wachstumsgrundlage zu dienen. Diese aufgeschlossenen Braunalgen werden mit pflanzlichen Aminosäuren, Salzen, Laugen, Nährstoffsalzen, Spurennährelemente, Tonmineralen in flüssiger, dickflüssiger und fester (granulierte oder pulver) Form den Biogas-Anlagen zugeführt und dienen als Wachstumsgrundlage für die Mikroorganismen bei der Biogas-Bildung in der Biogasanlage (feucht, nass oder trocken Fermenter).

Ein flüssiger Aufschluss mit Alkalimetallen und eine hohe Konzentration der Alkalimetalle führen mit Wasser zum totalen Aufschluss der Braunalgen, der für diese Zwecke auch möglich ist.

Die Wirksamkeit des verwendeten Algenmaterials kann durch den Vorgang eines Aufschließens wesentlich verbessert werden. Das Aufschließen des Algenmaterials, beispielsweise in Form von Algenmehl, Algenextrakten und/oder Algenpasten, erfolgt durch einen Kontakt des Algenmaterials mit einer Aufschluss-Substanz. Eine derartige Aufschluss-Substanz kann beispielsweise aus Natriumoxid, -hydroxid, carbonat und/oder anderen Salzen oder Kaliumoxid,-hydroxid, - carbonat und/oder anderen Salzen bzw. Alkalimetallen bestehen.

Vorzugsweise erfolgt ein derartiges Aufschließen in einem feuchten Zustand des Algenmaterials nach Wasserzusatz. Bevorzugt erfolgt das Aufschließen mit Verbindungen von Alkalimetallen oder Erdalkalimetallen, insbesondere von Kalium, Natrium, Ammonium und/oder Magnesium. Als Verbindungen kommen insbesondere Oxide, Hydroxide oder Carbonate in Frage. Als Ergebnis des Aufschließens wird vorzugsweise ein granuliertes Produkt erzeugt.

Der Vorgang des Aufschließens kann beispielsweise in Rührkesseln oder in rotierenden Trommeln erfolgen. Gedacht ist beispielsweise an die Verwendung von rotierenden Trommeln mit im Wesentlichen horizontal oder schräg verlaufender Drehachse. Der Prozess des Aufschließens wird vorzugsweise schonend mit mäßiger mechanischer Beanspruchung des Algenmaterials durchgeführt. In Abhängigkeit vom jeweiligen Aufschlußergebnis kann eine leichte Temperierung des Algenmaterials und somit eine Wärmezufuhr von Vorteil sein.

Als zweite Komponente können anorganische Stoffe verwendet werden. Tonminerale, wie Montmorillonit, Vermikullit, Kaolinit, Gesteinsmehle in der Kombination mit aufgeschlossenen oder nicht aufgeschlossenen Algen führen zur Verbesserung der Biogas- und Methangas-Bakterien. Es werden geringere Mengen an Tonminerale und an Algen für die bessere Aktivität der Bakterien in der Kombination benötigt.

Als dritte Komponente können ebenfalls anorganische Stoffe verwendet werden. Oxide, Hydroxide und Salze wie Kalium-, Natrium-, Kalzium-, Magnesium-, Eisen- und Ammonium-Salze dienen in der Kombination zur verbesserten Schwefel-Bindung und schützen die Motoren der Stromgeneratoren vor Schwefelsäurekorrosion und korrigieren den pH-Wert in der BiogasAnlage.

Auch als vierte Komponente kommen anorganische Stoffe zum Einsatz. Phosphat und Spurennährelemente (Zn, Cu, Mn, Co, Ni, Mo, Cl, Se, und andere) dient der Ausgleich beim Fehlen in organische Masse (z.B. bei Mais) in Fermenter.

Als fünfte Komponente werden organische Substanzen eingesetzt. Aminosäuren und oder Proteine (pflanzliche, tierische oder synthetische) verbessern die Zusammensetzung und die Aktivität der Mikroorganismen in Fermenter (in Biogasanlage).

Die Wirkung besteht insbesondere in einer Belebung der Biogasbakterien.

Die Inhaltsstoffe des im weiteren als Biogas-Algin bezeichneten Präparates sind aufgeschlossene Braunalgen (Ascophyllum nodosum), mikroskopisch kleine Alginatmoleküle, die ein kleines Festbett für Bakterien darstellen. Die Wirkung ist ähnlich wie bei einem Katalysator, der zur Belebung der Bakterien in org. Substanz führt. Alginate verhalten sich wie die Zündkerze im Benzinmotor. Kleine elektrische Funken bringen dort das Benzin-Luft-Gemisch zur Explosion. Auch diese kleine Alginat-Menge von 100 - 125 g Biogas-Algin ist in der Lage, 1000 Liter org. Substanz durch explosionsartige Vermehrung der Bakterien zum schnellen Abbau zu bewegen.

Die Symbiose zwischen acetogenen und methanogenen Bakterien und das biologisches Gleichgewicht bleiben erhalten, nur der Abbau der org. Substanz wird beschleunigt. Die Förderung der acetogenen und methanogenen Bakterien führt dazu, dass Essigsäure, Propionsäure u.a. org. Säuren abgebaut und einem "Umkippen" in Fermenter entgegengewirkt werden.

Mit Hilfe von Biogas-Algin BASS plus wird die Aktivität der Mikrobiologie in der Biogasanlage stark erhöht. Die biologischen Prozesse innerhalb des Fermenters laufen besser und schneller ab. Ein weiterer Effekt ist die Senkung der Schadgase Schwefel und Ammoniak. Schwefel und Ammoniak werden an Biogas-Algin BASS plus gebunden und aus dem Fermenter ausgetragen. Im Pflanzenbau stehen die Nährstoffe der Pflanze voll zu Verfügung.

Vorhandene Schwimmdecken werden durch den erhöhten Abbau der organische Trockensubstanz reduziert. Dadurch ist eine Steigerung der Raumbelastung sowie des Leistungspotentials der Biogasanlage möglich. Durch die Reduzierung der Schwimmdecke im Fermenter verringert sich der Energiebedarf der Rührwerke.

Der Sauerstoffeintrag kann verringert werden, weil der Schwefel an Biogas-Algin gebunden wird.

Die Wirksamkeit ergibt sich aus den nachfolgend erläuterten Versuchen. In den beigefügten Tabellen sind charakteristische Daten zu den nachfolgenden Beispielen zusammengefasst. Es zeigen im Einzelnen:
Die Wirkung der ersten Komponenten, insbesondere der ersten, zweiten, dritten, vierten und fünften Komponente: (aufgesch. Braunalgen+ Montmorillonit+ Kaliumcarbonat+Kalk+Eisen+ Aminosäuren) einzeln ist bekannt (jede einzelne aufgeführten Stoffe haben mit hohen Aufwandmengen eine spezifische Wirkung. Mischungen der Komponenten potenzieren die Wirkung und senken die Aufwandmenge der einzelnen Komponenten.

Einzelne Komponenten: Siehe Tab. 1: zwar zeigen die einzelnen Stoffe einen Effekt, jedoch reichen die Wirkungen bei weitem nicht aus.

Erste, zweite und dritte Komponente: aufgesch. Braunalgen + Montmorillonit, + (Kaliumcarbonat + Kalk +Eisen) (BASS) Erste, zweite, dritte und vierte Komponente: aufgesch. Braunalgen + Montmorillonit, + (Kaliumcarbonat + Kalk +Eisen)+ Aminosäuren (BASS Super)
Erste, zweite, dritte, vierte und fünfte Komponente: aufgesch. Braunalgen + Montmorillonit, + (Kaliumcarbonat + Kalk +Eisen) + Aminosäuren+ Spurenelemente (BASS Super plus)
+ Spurennährelemente ist nicht bekannt.
Erste und zweite Komponente: aufgesch. Braunalgen + Montmorillonit, (BAS)
Siehe Tab. 1: der Effekt der Mischungen BAS, BASS, BASS Super und BASS Super plus verbessert sich gegenüber der Wirkung der einzelnen Komponenten und bei vollständiger Zusammensetzung der Komponenten in BASS Super plus wird die Methangas-Ausbeute am besten, weil auch die fehlenden essentiellen Aminosäuren, Polysaccharide, Melasse oder Vinasse und Spurennährelemente ergänzt werden.

### Resultate:

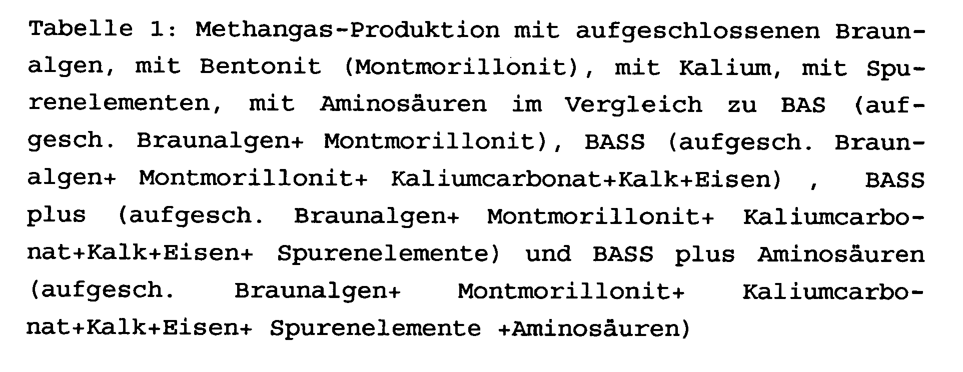

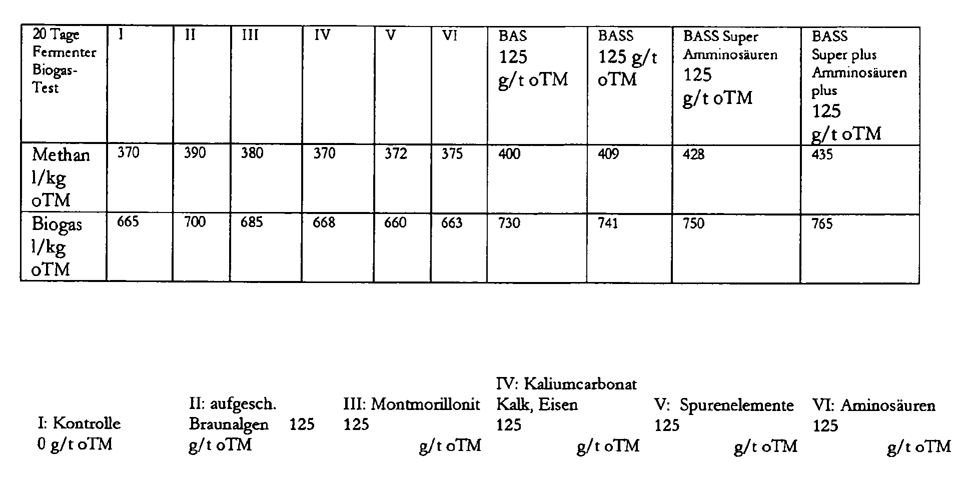

Ergebnisse in den Tabellen 2 und 3 zeigen die rasante Entwicklung der Bakterien und Pilze nach der Behandlung mit BAS (aufgesch. Braunalgen + Montmorillonit im Vergleich zu unbehandelt. Je schneller und desto besser die Entwicklung der Mikroorganismen, um so besser die Methangasbildung.

**Tabelle 2: Bakterienentwicklung mit Biogas-Algin in Vergleich zu unbehandelt**

| Bakterien-Population | 1 Std. | 2 Std. | 8 Std. | 24 Std. | 36 Std. |
|---|---|---|---|---|---|
| **Kontrolle Nährlösung** | 10,00 | 10 | 1000000 | 1000000 | 10000000 |
| **Biogas-Algin (BAS)** | 10000 | 1000000 | 100000000 | 100000000 | 10000000 |

**Tabelle 3: Pilzentwicklung mit Biogas-Algin in Vergleich zu unbehandelt**

| | | | | | |
|---|---|---|---|---|---|
| Pilz-Kolonien | 1 Std. | 2 Std. | 8 Std. | 24 Std. | 36 Std. |
| Kontrolle Nährlösung | 0,00 | 0 | 1 | 1 | 10 |
| Biogas-Algin (BAS) | 0 | 1 | 2 | 4 | 10 |

Die Tabellen 4, 5 und 6 zeigen der Anstieg der aeroben und anaeroben Bakterien und die Bindung von SH2 sowie NH4 mit der BASS (aufgesch. Braunalgen + Montmorillonit, + (Kaliumcarbonat + Kalk +Eisen) (BASS) eindeutig gegenüber der Kontrolle.

**Tabelle 4: Aerobe und anaerobe Bakterienentwicklung mit Biogas-Algin (BASS) in Vergleich zu unbehandelt**

| Produkt | Gesamtkeimzahl g/Frischsubstanz | |
|---|---|---|
| | aerob | anaerob |
| **Start** | 2600 | 0 |
| **Ende Kontrolle** | 4000 | 15000 |
| **Biogas-Algin BASS** | 14000 | 40000 |

**Tabelle 5: H2S-Senkung mit Biogas-Algin (BASS) in Vergleich zu unbehandelt**

| | **Kontrolle 0,4 g Biogas-Algin** | |
|---|---|---|
| | SH₂ ppm | (BASS) SH₂ ppm |
| **3 Wochen Lagerung** | 438 | 116 |
| **5 Wochen Lagerung** | 483 | 178 |

**Tabelle 6: Ammoniumfixierung durch Biogasalgen im Vergleich zu unbehandelt**

| | Unbehandelt % | Biogas-Algin BASS % Unbehandelt = 100 % |
|---|---|---|
| **N-Gesamt** | 100 | 94,7 |
| **NH4-N** | 100 | 76,7 |

Die Tabelle 7 zeigt die Wirkung der Mischung [aufgesch. Braunalgen + Montmorillonit, + (Kaliumcarbonat + Kalk +Eisen) + Aminosäuren+ Spurenelemente (BASS Super plus)] in FM (Feucht- 1, 2, 3 und Trocken-Fermenter und im Perkulat (behandlung vom 20.11.2006-23.01.2007). Ein kontinuierlicher Abfall der H2S-Gehalt ist festzustellen.

**Tabelle 7: H2S-Gehalt durch Biogas-Algin (BASS Super plus) Behandlung der Biogasanlage Trocken- und Feuchtfermenter 2006 in der Praxis.**

| | FM1 | FM2 | FM3 | Perkulat | Nass FM |
|---|---|---|---|---|---|
| 20.11-12.12 | 308 | 321 | 297 | 299 | 166 |
| 13.12-31.12 | 205 | 214 | 185 | 230 | 174 |
| 1.1-9.1 | 155 | 167 | 211 | 184 | 192 |
| 10.1-23.1 | 129 | 158 | 172 | 186 | 202 |

### Biogas-Algin Wirkung:

Schwermetalle, wie Kupfer, Eisen und andere werden durch Biogas-Algin (als Ionen-Austauscher) gebunden und deren Giftigkeit gegenüber Bakterien verhindert.

## Patentansprüche

1. Präparat zur Optimierung der Methangasbildung in Biogasanlagen, das mindestens eine erste organische Komponente sowie mindestens eine zweite anorganische Komponente aufweist, **dadurch gekennzeichnet, daß** die erste Komponente aus Algenmaterial besteht.

2. Präparat nach Anspruch 1, **dadurch gekennzeichnet, daß** die organische Komponente aus aufgeschlossenen Algen besteht.

3. Präparat nach Anspruch 1 oder 2, **dadurch gekennzeichnet, daß** die erste Komponente aus Braunalgen besteht.

4. Präparat nach einem der Ansprüche 1 bis 3, **dadurch gekennzeichnet, daß** als weitere organische Komponente mindestens eine Aminosäure enthalten ist.

5. Präparat nach einem der Ansprüche 1 bis 3, **dadurch gekennzeichnet, daß** als weitere organische Komponente mindestens ein Vitamin enthalten ist.

6. Präparat nach einem der Ansprüche 1 bis 3, **dadurch gekennzeichnet, daß** als weitere organische Komponente mindestens ein Hormon enthalten ist.

7. Präparat nach einem der Ansprüche 1 bis 3, **dadurch gekennzeichnet, daß** als weitere organische Komponente mindestens ein Laminarin enthalten ist.

8. Präparat nach einem der Ansprüche 1 bis 3, **dadurch gekennzeichnet, daß** als weitere organische Komponente mindestens ein Fokuzin enthalten ist.

9. Präparat nach einem der Ansprüche 1 bis 3, **dadurch gekennzeichnet, daß** als weitere organische Komponente mindestens ein Betain enthalten ist.

10. Präparat nach einem der Ansprüche 1 bis 9, **dadurch gekennzeichnet, daß** als zweite anorganische Komponente ein Tonmineral zugesetzt ist.

11. Präparat nach einem der Ansprüche 1 bis 9, **dadurch gekennzeichnet, daß** als zweite anorganische Komponente ein Betonit zugesetzt ist.

12. Präparat nach einem der Ansprüche 1 bis 9, **dadurch gekennzeichnet, daß** als zweite anorganische Komponente ein Spurenelement zugesetzt ist.
